# EUROPEAN PATENT SPECIFICATION

(11) **EP 1 536 781 B1**
(45) Date of publication and mention of the grant of the patent: **17.02.2010**
(21) Application number: 03760967.4
(22) Date of filing: 19.06.2003
(51) Int. Cl.: A61K 31/195, A23L 1/305

(54) **NUTRITIONAL OR PHARMACEUTICAL COMPOSITIONS FOR INCREASING THE CREATINE RESPONSE OF ORGANISMS**
NAHRUNGS- ODER PHARMAZEUTISCHE ZUSAMMENSETZUNGEN ZUR ERHÖHUNG DER CREATIN-ANTWORT VON ORGANISMEN
COMPOSITIONS NUTRITIONNELLES OU PHARMACEUTIQUES POUR AUGMENTER LA REPONSE CREATINIQUE D'ORGANISMES

(30) Priority: 19.06.2002 EP 02077434
(43) Date of publication of application: 08.06.2005
(73) Proprietor: N.V. Nutricia, 2700 MA Zoetermeer (NL)
(72) Inventor: HAGEMAN, Robert, Johan, Joseph, NL-6705 CT Wageningen (NL); VERLAAN, George, NL-6708 SB Wageningen (NL)
(74) Representative: van Westenbrugge, Andries
(86) International application number: PCT/NL2003/000448
(87) International publication number: WO 2004/000297

(56) References cited:
- EP-A- 0 532 369
- WO-A-91/07954
- GB-A- 696 405
- US-A- 4 871 550
- US-A- 5 767 159

## Description

### FIELD OF THE INVENTION

The present invention relates to a nutritional or pharmaceutical composition comprising a L-serine containing-component and an energy metabolism precursor wherein the composition is substantially free of glycine. A process for making such composition is herein provided as well as the use of the composition for increasing the creatine response and the methylation reaction capacity of organisms.

### BACKGROUND OF THE INVENTION

Adenosine triphosphate (ATP) is the immediate source of energy for allowing many processes in the mammals body such as biosynthetic and metabolic pathways, for maintaining ionic gradients and transport over membranes and for muscle contraction. ATP is metabolised in the muscle by the cleaving of one phosphoryl group. The chemical energy produced from this group can then be used to contract the muscle. As a result, adenosine diphosphate (ADP) is produced as a by-product. ATP can be produced in the muscle from glycogen or phosphocreatin. Phosphocreatine provides a ready source of energy-rich phosphoryl groups and is able to resynthetise ATP at nearly twice the rate compared to glycogen.

However, the amount of ATP in the muscle is small. Therefore a reserve supply of readily available energy is needed.

Phosphocreatine in muscle cells serves as a reservoir of high potential phosphoryl group. Creatine enters the muscle tissue by active transport where it can be converted to phospho-creatine by the action of mitochondrial or cytosolic kinases. The reversible transfer of a phosphoryl group from creatine phosphate to ADP to form ATP keeps the intracellular ratio of the amounts of ATP to ADP high. Muscle fatigue and the accumulation of lactic acid occur in energy-deficient states.

Creatine is a nitrogenous acid distributed in the mammals body in many tissues, e.g. in muscular tissue, but also in liver, pancreas, brain, retina, testes and kidneys. It is also called N-methyl-N-guanylglycine. Creatine is synthesised in the body through the processing of the amino acids glycine, arginine, and methionine and is supplied to the body by the food intake.

Creatine is available from many food sources and can be found in the muscle of most mammals and fish, including beef, chicken, cod, herring, pork, salmon, tuna and turkey. However, a problem encountered with the supplying of creatine via cooked food is that the cooking process tends to deteriorate the creatine. Further, most of the high creatine-containing food are also high in fat and cholesterol, thus minimising the interest for high intake of creatine via food.

Still, the supply of creatine to the body to enhance physical performance has become a popular habit as a substitute for steroids and other drugs in various sport and body building regimes. Indeed, because creatine is an amino acid normally biosynthesised in vertebrates, it is not included in the list of components that are prohibited for use as published by the International Olympic Committee, which list includes more than 120 kinds of products.

Accordingly, creatine supplements have become a popular source of obtaining the nutrient, especially for the body-building industry, e.g to increase lean body mass and among athletes e.g to increase working capacity.

Another category of persons to which the supply of creatine supplements is beneficial are those persons where a high demand is put on the body to provide sufficient energy, i.e. ATP. These include, but are not limited to, persons where the blood supply has been imparted such as after a trauma, disease affected persons, or even vegetarians people.

The use of creatine in the production of supplements is widely known, especially in the area of sport.

Hence, US 5,973,005 discloses a drink comprising an aqueous solution of creatine, wherein the creatine is under the form of creatine acid sulfate, for providing a source of creatine to an animal in need thereof.

US 6,136,339 discloses the combination of creatine with lipoic acid for enhancing an athlete muscle's size or strength.

US 6,172,114 discloses the combination of creatine with ribose for increasing muscle strength while reducing the side effect linked with creatine, namely diarrhea and nausea.

Creatine containing supplements are also discussed by Gregory S. Kelly, N.D in Alt. Med Rev. 1997; 2(3):184-201. In this article, a supplement comprising creatine monohydrate, protein shake, vitamin C, and a multivitamin/mineral formulation is described as providing best result on the athletes strength.

However, a problem associated with the use of creatine is that it is believed that in order to provide a significative response of creatine within the mammals muscle, a relatively large dose of creatine (2 to 20g per day) has to be administered to the mammals. This high dosage can impart the normal dietetic patterns and induces diarrhea or even nausea. Further, it was also reported in Stead L.M. et al, Am J Physiol, 281, E1095, 2001, that consumption of large amounts of creatine by rats, taking a dosage that is equivalent to a dose of 20g creatine for a subject of 70 kg per day, i.e. 0.28g of creatine per kilo and per day, could lead to a decrease in the capacity of endogenous creatine biosynthesis due to a decrease in the expression of L-arginin:glycine amidinotransferase. However, decreasing the capacity of endogenous creatine biosynthesis is undesirable, especially when specific high demands on methylation capacity are required like during diseases or surgery. Accordingly, one solution known in the art to remediate to this decrease is to discontinue the usage of creatine for a certain period on a periodic basis.

In addition, creatine is unstable in aqueous solution, and tends to form creatinine when present in acidic conditions. Creatinine is the inactive form of creatine and is quickly excreted from the body. Creatinine cannot be converted into phosphocreatine and does not participate in the regeneration of ATP. Consumption of creatinine may complicate the interpretation of the increase of creatinine levels due to disease such as kidney disorders.

Accordingly, there is a need for a composition that provides an effective creatine response in the mammals muscle without causing the side effects linked to the use of creatine or having the potential safety problems linked with the uncontrolled use of creatine.

One solution to this problem is to use an energy metabolism precursor of guanidino-acetic acid (GA) type or also called glycocyamine or N-amidinoglycine in the literature.

In mammals, GA is a natural precursor of creatine and is produced from arginine by transfer of its amidino group to glycine, thereby resulting in the formation of GA. GA can then be converted into creatine by methylation with a methyl transferase present in many tissues of the mammals, which uses S-adenosyl methionine (SAM) as a substrate.

Use of GA in therapeutic composition is known from US 2,761,807 but is also known from commercially available sport nutrition supplements.

US 2,761,807 discloses the combination of GA with a material selected from betaine, betaine hydrate, choline, and dimethylthetin for providing a source of energy in the treatment of cardiovascular diseases, paresis resulting from poliomyelitis, multiple sclerosis, and the anxiety-tension-fatigue syndrome. The described amount of GA to be administered is of 30 mg GA per pound body weight per day, which results in the consumption of about 4.6-6 g GA per day, dependent on the body weight. The material selected from betaine, betaine hydrate, choline, and dimethylthetin are to be included in a 3-5 fold molar excess compared to GA, thus, taking betaine as example in 4 fold molar excess, resulting in the consumption of 20 g of betaine. This results in the consumption of a high amount of a composition of about 25 g of powder per day, which appears burdensome in a conventional dietetic pattern especially when the taste of the active components itself is not to be appreciated. This problem of undesirable taste is even more acute among athletes, who are regular users of such compositions. One solution to minimise the undesired taste is by mixing with other ingredients or by dissolution in liquid at for example a 5% dissolution rate. However, dissolution would then require them to drink a volume of at least 500 ml per day.

Sport nutrition supplements are also available on the market. For example, Syntrax Innovations markets Swole as a product for non-creatine responders. This product contains per serving of 7g: 1.5g GA, 2.5 g glucuronolactone, 2 g creatine and 0.5 dimethylglycine. Two serving per day are recommended. This results in the consumption of 14 g dry powder of the supplement composition, which in turns requires drinking 250 ml of diluted supplement composition. Further, the supplement uses glucoronolactone, which is in itself an expensive component, thereby increasing the cost of the resulting composition. Again, this supplement also comprises unpalatable components, i.e. components with an undesired taste.

It has been found that reduction of the amount of unpalatable components within supplement compositions should preferably be lower than 10 g, and preferably below 6g on a daily basis to provide a more desirable taste.

Further, consumption of high doses of GA alone has been reported as being potentially causing epileptic seizures, especially with patients with inherited decrease of the GAMT expression. Consumption of high doses of GA alone has also been associated with undesired side effects, which may be elated to the accumulation of GA, thus causing a distortion in the creatine, methylation and energy metabolism.

Still, persons suffering from a metabolic stress such as the ones which occur after surgery, traumata, malnutrition or in catabolic states encounter a problem with the capacity of methylation reaction of their organism. Rapidly proliferating or differentiating tissues such as epithelial cells, e.g. of the gastrointestinal tract, bone marrow, endo- or exocrine tissues such as some tissues in the gonades and pancreas are particularly sensitive to metabolic stress, thus hindering or reducing the capacity of the organism of methylation reactions.

Insufficient methylation capacity can be detected by measuring the capacity of a tissue to generate SAM per time unit. It is the inventor's belief that the presence of homocystein in the blood plasma after an overnight fasting represents an indicator of decreased methylation capacity. Hence, it has been found that large groups of persons suffer in their daily life from such elevated homocystein levels. High levels of homocystein in the blood plasma can also arise from disorders in the organs or from disease states.

Nevertheless, in mammals, methylation of components is an important process reaction. Indeed, as described hereinbefore, GA is converted into creatine by a methylation reaction.

Accordingly, there is a need for a composition that is effective in decreasing homocystein levels, shows no or reduced occurrences of side effects due to the use of the composition while still exhibiting a beneficial effect on creatine status in tissue.

It is also an object of the invention to produce a composition that has one or more of the following objectives: good taste, reduced cost, and/or that do not require, if the composition used as a supplement is diluted, to drink a high amount of the diluted composition, i.e. preferably no more than 150 ml, most preferably no more than 100 ml.

It has now advantageously been found that the use of a protein component or fraction thereof that comprises a high level of serine contained therein fulfils such a need and further acts as effective aid for the metabolism of energy.

It has further been found that the combination of such protein fraction containing L-serine with an energy metabolism precursor of the GA type was also effective in producing an effective creatine response to the mammals muscle without producing the side effect of creatine.

Not to be bound by theory, it is believed that L-serine induces the methylation of GA or equivalents thereof via a series of reaction ending in the methylation of GA into creatine.

The use of L-serine, in particular in high amount, to improve health and/or to reduce the side effects of the energy metabolism precursor and/or to increase the methylation capcity of organisms is not recognised in the art. To the contrary, in mammals, serine is considered as a non essential amino-acid for nutrition or pharmaceutical compositions. Hence, Gregory S. Kelly, N.D in Alt. Med Rev. 1997; 2(3):184-201 prescribed the use of protein, in particular whey protein to provide the sufficient levels of amino acid, mainly sulfur amino acid (methionine and cysteine), branched amino acid (leucine, isoleucine and valine) but also glutamine and glycine. Proteins always contain serine and glycine. The table below gives the weight ratio associated with various type of proteins:

| | Weight ratio of serine: Glycine |
|---|---|
| Whey proteins isolated from bovine milk | about 2.5 |
| soy protein | about 1.2 |
| organ meat | 0.1-1 |
| total chicken egg | 2.2 |
| chicken egg yolk | 2.6 |
| vegetable protein | 0.2-1.5 |

Thus, it can be said that serine is not considered to be an important dietetic amino acid. Instead, it is either considered to be sufficiently quantitatively present in the dietetic protein, i.e. provided by food intake, or that the presence of glycine and alanine as part of the dietetic protein can ensure sufficient serine biosynthesis. However, it has now been found that the presence of a high level of glycine hinders the health promoting action of the serine. Indeed, glycine which acts as a methyl acceptor interacts with serine which acts as a methyl donor.

### SUMMARY OF THE INVENTION

There is provided a nutritional or pharmaceutical composition comprising:
a)-a protein fraction containing L-serine selected from the group consisting of L-serine, protein or peptides; and
b)-an energy metabolism precursor selected from glycocyamine (GA), equivalents thereof, and mixtures thereof, by equivalents thereof, it is meant:
   - compounds which provide GA in blood plasma after oral consumption and which are selected from guanidino-acetic acid salts of sodium, potassium, calcium, ammonium, magnesium, zinc, iron, copper, chromium and mixtures thereof and/or GA bidentate with zinc to a maximum of 100mg zinc per daily dose of the total composition,
   - ester and/or ether compounds that can be metabolised to guanidino acetate in the gut or body, and compounds where the amidino group of the GA is modified by protonation and thus forms a salt,
wherein the composition is free of glycine or if glycine is present within the composition, the weight ratio of L-serine to glycine after hydrolysis of the composition is of more than 2.7 to 1.

In another aspect of the invention, there is provided a process for making said composition.

In a further aspect of the invention, there is provided the use of the composition for increasing the creatine response within mammals muscle and increasing the capacity of methylation reaction of the organism.

Still, in another aspect of the invention is provided the use of the protein fraction containing L-serine for the preparation of nutrition or pharmaceutical composition for increasing the creatine response and methylation capacity of the organism, wherein the protein fraction containing L-serine comprises no glycine or if present the weight ratio of L-serine: glycine after hydrolysis of the protein containing L-serine is of more than 2.7:1.

It is also believed that the presence of the protein fraction containing L-serine is beneficial to the effect linked to the energy metabolism precursor of the GA type on the enzyme described in Am. J. Physiol. Endocrinol. Metab., Nov. 2001, 281 (5): E1095-E1100, and thus beneficial to health. Indeed, the increase in methionine synthase increases in turn the methylation capacity of the organism but only on the condition that sufficient substrate is present. It has been found that a substrate containing a high level of serine, with preferably sufficient levels of co-factors like vitamin B6, folic acid, vitamin B12 and zinc is an effective substrate.

Further, it is also believed that by the present invention, an increase in cysthathione synthase is obtained, which provides an increase in the endogenous cysteine production, and in turn provides under certain conditions an increase in the glutathion biosynthesis and sulphation capacity.

### DESCRIPTION OF THE INVENTION

### Protein fraction containing L-serine

A protein fraction containing L-serine is an essential component of the invention. For the purpose of the invention, a protein fraction containing L-serine has been found advantageous for increasing the methylation capacity of the organism, thereby increasing the creatine response. However, to provide this effect, the protein fraction containing L-serine, in particular when incorporated in nutrition or pharmaceutical composition, needs to be present within the composition either in the absence of glycine or if glycine is present within the L-serine containing-component and/or final composition, it is then required that the weight ratio of L-serine: glycine after hydrolysis of the final product is of more than 2.7 to 1.

L-serine, also called serine herein, can endogeneously be formed by transamination of hydroxypyruvate but can also be formed from glycine, e.g. the dietetic glycine or the glycine that is formed from choline degradation, via betaine, dimethylglycine and sarcosine. L-serine is also present in proteins. Functional equivalents can also be used herein in addition or in place of the L-serine.

By "protein fraction containing L-serine" it is meant protein containing L-serine but also L-serine containing component selected from L-serine, equivalents thereof and mixtures thereof. By "equivalents", it is meant L-serine in its zwitterion form, L-serine in its salt form, optionally coupled to the amino or carboxy group, peptides, proteins, and mixtures thereof. For the purpose of clarity, phosphatidylserine is not considered as a suitable "equivalent".

The requirements for the peptides and protein to be used as L-serine containing components are that:
1)-they can be digested in the total gastrointestinal tract to form serine or can provide L-serine after metabolism of the peptides in the liver, and
2)-they provide a weight ratio of L-serine to glycine after hydrolysis of the final product of more than 2.7:1

As described herein before, it is essential for an effective methylation capacity that the weight ratio of L-serine to glycine within the proteinaceous component, if used alone, or if incorporated in the composition comprising the energy metabolism precursor is of more than 2.7:1, preferably of at least 2.9:1, more preferably of least 3.1:1, and most preferably of at least 3.4:1, such as in the range of 3.7:1 to 10.000:1. It is also preferred that the total doses of serine is not too high because this may impart normal dietetic practices. In a supplement the weight ratio of L-Serine to glycine can be relatively high because serine can be the only amino acid that is included in the preparation. However in a complete composition also glycine will be present which causes the ratio to be typically below 6:1 and preferably below 4:1.

Food grade qualities of L-serine are commercially available from Degussa and other amino acid manufacturers. Proteins such as caseinates from milk, or egg proteins are also readily available.

Levels within the composition will depend on type of product. Nutritional supplements will typically comprise 25-90 wt.% protein component.

### Dosage:

When the protein fraction containing L-serine is used in a nutritional supplement, a dose of at least 1g per day should be administered so that at least 0.75g excess L-serine is consumed compared to glycine, preferably a dose of at least 1.2g L-serine so that at least 1g excess L-serine is consumed compared to glycine, and more preferably a dose of at least 1.8g L-serine so that at least 1.5g excess L-serine is consumed compared to glycine. Preferably, the daily dosage amounts to up to 30 g, more preferably up to 15 g.

When the protein fraction containing L-serine is used in a complete nutrition composition, a higher dose of L-serine will be used, i.e. of at least 4.8g per day should be administered so that at least 3.2g excess L-serine is consumed compared to glycine, preferably a dose of at least 5.5g L-serine so that at least 3.9g excess L-serine is consumed compared to glycine, and more preferably a dose of at least 6.4g L-serine so that at least 4.7g excess L-serine is consumed compared to glycine. Preferably, the daily dosage amounts to up to 30g, more preferably up to 20 g.

By "glycine", it is meant the amino acid per se. Sarcosine or dimethylglycine are not included by the term "glycine". Accordingly, when the weight ratio of serine to glycine has to be determined for a specific peptide, protein, or mixture of amino acids with proteins or peptides, the hydrolysis of the final product or mixture will have to be performed. A suitable method of determination of the L-serine:glycine weight ratio for use herein is given in The AOAC Official methods of Analysis 1984, nr 43.263 and 43.264.

Still another suitable method of determination of the L-serine:glycine weight ratio for use herein is by calculation from published standard and recognised values.

It is further preferred to include a relatively low level of guanidino compounds other than GA or equivalent thereof and creatine in the nutritional compositions. These compounds are arginine or citrulline or ornithine or their functional equivalents such as salts. These compounds impart a further creatine response of the GA and creatine in the product. Typically serine levels in the protein component, when these component are present are then more than 1.7 times and preferably above 1.8 and most preferably above 2.2 that of the levels of arginine or citrulline or ornithine.

Preferably, ornithine is absent from the composition.

The amount of GA or equivalent thereof should therefore be of at least 0.3 times the total amount of other guanidino compounds and preferably at least 0.5 times the amount of arginine or citrulline or ornithine.

It is further preferred to include a minimum amount of L-methionine in the composition of at least 0.5 g per daily dose, and preferably more than 0.8 g. L-methionine can be present as pure amino acid, its salt or as peptide or protein.
It is also preferred to include L-histidine in an amount of at least 0.5 and preferably more than 0.8 g per daily dosis. L-His can be present as pure amino acid, as salt, or as part of a peptide or protein.

### Energy metabolism precursor

The energy metabolism precursor for the purpose of the invention is selected from guanidino-acetic acid (GA), equivalents thereof, and mixtures thereof. GA is a natural energy metabolism precursor of creatine. GA results from the transfer of arginine's amidino group to glycine, which in turn gives GA.

For the purpose of the invention, GA or equivalent thereof are used as energy metabolism precursors. Accordingly, GA can be used in its pure acid form.

By "equivalents thereof", it is meant:
- compounds which would provide GA in blood plasma after oral consumption and which are preferably selected from guanidino-acetic acid salts of sodium, potassium, calcium, ammonium, magnesium, zinc, iron, copper, chromium and mixtures thereof and/or GA bidentate with zinc to a maximum of 100mg zinc per daily dose of the total composition,
- ester and/or ether compounds that can be metabolised to guanidino acetate in the gut or body, e.g. by hydrolysis, i.e. preferably esters of the carboxy group with lower organic acids and selected from esters of acetic acids, esters of propionic acid, esters of butyric acid, and mixtures thereof. Advantageously, these esters are stable in solution, thus making them suitable for incorporation into liquids, in particular drinks or for enteral administration.

Further suitable compounds as "equivalents" are compounds where the amidino group of the GA is modified by protonation and thus forms a salt with, for example, anions that are commonly used in food manufacture, such as chloride, bicarbonate, and hydroxide. For the purpose of the invention, it is preferred not to use anions that might promote nitrite formation in vivo, such as nitrate, nitrite, or sulphite.

### Dosage:

For the purpose of the invention, the amount of "energy metabolism precursor" to be employed in the invention of the composition is linked to the excess of L-serine versus glycine. Accordingly, on a molar basis, it is preferred that the molar amount of "energy metabolism precursor" to be daily administered lies within the range of from 0.1-10 times the excess of L-serine versus glycine, and more preferably lies within the range of from 0.2-4 times the excess of L-serine versus glycine.

Stated otherwise in terms of daily dosage per se of "energy metabolism precursor", it is preferred that the "energy metabolism precursor" is administered to mammals at a low dosage per day of at least 0.2g, preferably at least 0.5g, more preferably at least 0.7g, and up to 4g, preferably up to 3g. In particular, during chronic use, a low daily dosage of energy metabolism precursor between 0.8-3g was found effective to produce an effective and safe creatine response from the "energy metabolism precursor".

### Optionals

The composition of the invention may also comprise optionals which will further secure an adequate methylation capacity of the organism. Preferred optionals for use herein are selected from creatine, vitamins, carbohydrates, aldehydes, mineral, and mixtures thereof.

### Creatine

Addition of creatine to the invention composition has been found advantageous. Indeed, it has been found that by addition of creatine to the invention composition, the creatine response was increased without causing the known side effects of creatine.

Creatine, when present, can be used as it is and/or in its salt form and/or in its hydrate form. Suitable salts of creatine for use herein are those selected from salts of creatine with inorganic acid (preferably creatine phosphate, and/or creatine chloride), salts of creatine with organic acid (preferably creatine citrate and/or creatine pyruvate and/or creatine malate and/or creatine fumarate and/or creatine isocitrate). For the purpose of the invention, it is preferred to use salts of creatine with organic acid. The creatine, when present, is present in weight ratio of energy metabolism precursor:creatine of from 0.2:5, preferably from 0.6:4.5, more preferably from 1.1:4, and most preferably from 1.2:3.6.

### Vitamins

Vitamins are also preferred optional for the purpose of the present invention. Indeed, it has been found that addition of vitamin, in particular those selected from vitamin B6, vitamin B12, folic acid, and mixtures thereof, in a doses above twice the RDA during several days (e.g. 5 days) produces a further increase in the creatine response to most of the hospital patients that need an anabolic response.

### Carbohydrate

The composition of the invention can optionally comprises a digestable food grade carbohydrate. A suitable carbohydrate for use herein are glucose sources such as maltodextrin.

### Minerals

Minerals can also be advantageously used in the invention composition. Suitable minerals for use herein are selected from chromium, vanadium, selenium, magnesium, boron, zinc, potassium, sodium and mixtures thereof, preferably magnesium and/or zinc. When present, the amount of iron in the composition is preferably present at a low amount, preferably of less than 3 mg per daily dose and more preferably of less than 30 mg per daily dose.
When potassium and sodium are present, the molar ratio of potassium to sodium is preferably above 1.

The composition of the invention may further optionally comprises minors such as natural and/or artificial flavoring components, dyes or other coloring additives, preservatives, lubricants, binders, and fillers. Glycerol may also added to the invention composition to improve the absorption of the "energy metabolism precursor" and faciliate consumption.

### Form of the composition

The composition of the invention may be in any form suitable for its administration whether orally or enteral administration. Typical forms suitable for use herein include liquid form, powder form, emulsion form, suspension form, gel form, bar form, but also cookies or sweeties.

### Use/applications

There is provided the use of a composition of the invention for increasing the methylation reaction capacity within mammals organisms, which in turns increases the creatine response within mammals muscle.

Administration of the invention composition has been found beneficial to diseases affected persons, such as hospitalised persons, persons suffering from mild health problems or who want to prevent a further worsening of their condition, in particular when suffering from cardiovascular, cerebrovascular, ischaemic diseases or disorders associated with problems with creatine metabolism or energy supply but also vegetarian persons, elderly persons, infants, in particular those having small birth weight and thus having an anabolic reaction, athletes including also persons that require an increase in lean body mass.

In particular, administration of the invention composition is beneficial for:
- the increase of anabolic processes as is strongly desired in infants, especially those of low birth weight;
- providing an increase of the lean body mass,
- prevention and/or treatment of muscle catabolism or even cachexia as may occur after surgery, after traumata, during cardiovascular disease, with pancreas disorders, tumors, like those in liver, pancreas lung and kidney, during malnutrition, during several neurological disorders, during lung emphysema and other respiratory diseases, liver disorders like cirrhosis, severe inflammation like during inflammatory bowel disease, pancreatitis, hepatitis, AIDS and during severe insulin resistance as may occur in diabetes;
- the improvement of the energy status of tissues and cells, which will improve results during intermittent exercise, and is important during ischaemia/ reperfusion situations.

Advantageously, the increase of lean body mass and energy status leads to an increase in muscle strength and power. In addition vegetarians will profit from the invention composition since the presence of creatine is for a majority of vegetarians marginal.

Accordingly, there is also provided the use of the invention composition for the stimulation and/or increase of anabolic processes and/or the providing of an increase of the lean body mass, and/or prevention and/or treatment of muscle catabolism or even cachexia and/or for the improvement of the energy status of tissues and cells.

In addition, when the methyl donors, like the protein fraction containing L-serine according to the invention, are in excess of the methyl acceptor, like the GA or equivalents thereof, the invention composition has also been found beneficial for the prevention and/or treatment, in particular prevention of disorders selected from:
- cancer, especially cancer to liver, kidneys, pancreas, testis, mammae, ovaria and prostate and colon, but also by prevention of the occurance of hyperproliferation in tissue like gut (polyps), skin (warts) or prostate,
- neurological disorders such as Alzheimer's disease or other dementias, Parkinson's disease, sensitivity to pain, depression, multiple sclerosis and epilepsy,
- migraine, allergy, insulin resistance which improves glucose tolerance and decreases side effects of diabetes type II,
- cardiovascular and cerebrovascular disorders such as those described in WO 01/84961, hypercholesterolaemia, hypertension, prevention of hearing loss,
- subfertility,
- uncontrolled inflammation processes such as during inflammatory bowel diseases (e.g. Crohn's disease), pneumonia, but also by providing improvement of wound healing and especially decubitus, improvement of gut barrier function and prevention of sepsis; and
- mixture thereof.

By "excess methylation power", it is meant that the molar amount of methyl donors exceeds the molar amount of methyl acceptors, and preferably the amount of methyl donors minus methyl acceptors exceeds the average requirement for methyl groups per day as per given hereinbefore under dosage.

Accordingly, there is provided the use of invention composition when the methyl donor are present in excess of the methyl acceptor for the prevention and/or treatment, in particular prevention of disorders selected from cancer, neurological disorders, migraine, allergy, insulin resistance which improves glucose tolerance and decreases side effects of diabetes type II, cardiovascular and cerebrovascular disorders, hypercholesterolaemia, hypertension, prevention of hearing loss, subfertility, uncontrolled inflammation processes, pneumonia, and mixtures thereof, and/or improvement of wound healing, and/or improvement of gut barrier function and/or prevention of sepsis.

The following is a list with abbreviations that are used in the examples:

| Abbreviations | Definition |
|---|---|
| GA | Guanidino-acetic acid |
| Creatine Pyruvate | Creatine Pyruvate |
| vitamin premix 1 | vitamin premix on maltodextrin basis providing 200µg folic acid, 2µg cyanocobalamine and 2 mg vitamin pyridoxine |
| Milk protein | Milk protein providing about 0.48g L-serine and 0.16g glycine |
| vitamin premix 2 | vitamin premix providing 400µg folic acid monoglutamate, 1µg cyanocobalamine and 3 mg vitamin pyridoxine |
| Carbohydrate 3 | maltodextrin |
| Lipids | Mixtures of vegetable oils or marine oils as |
| Vitamin/mineral premix | vitamin mineral premix providing 30µg folic acid, 0.2 mg Vitamin B6 and 0.3µg vitamin B12 on maltodextrin basis, 1/20th of the recommended daily allowances for male adults for vitamin A, D, E, K, C, B1, B2, B3, calcium, phosphorus, Magnesium, zinc, iodine and selenium as published in the 10th edition of the Recommended Dietary Allowances as published by the US National Research Council, sodium 80 mg, Potassium 135 mg, chloride 125 mg, copper 0.15 mg, manganese 0.3 mg, pantothenic acid 0.4 mg, biotine 10 µg and choline 20 mg |

The following are non-limiting examples illustrating the invention.

### Example 1

Powder packed in sachet of 5g and comprising:

| Component | Levels |
|---|---|
| GA | 1.5g |
| Creatine pyruvate | 0.5g |
| L-serine | 2g |
| Vitamin premix 1 | 1g |

The powder contained in the sachet is meant to be dissolved in a suitable liquid such as water, juice, milk, pudding, or sauce for convenient administration.

### Example 2

Bar comprising per 25g:

| Component | Levels |
|---|---|
| Carbohydrate | 12g |
| Milk protein | 8g |
| L-serine | 0.6g |
| Tricreatine | 0.5g |
| GA | 0.5g |
| Vitamin premix 2 | 1 g |
| Moisture | up to 25g |

### Example 3

Complete nutrition composition in powder form for an hospitalised patient:

| Component | Levels |
|---|---|
| Caseinate | 4.0g |
| L-serine | 0.1g |
| GA | 0.1g |
| Creatine pyruvate | 0.1g |
| carbohydrate 3 | 11.3 |
| Lipids | 3.9g |
| Vitamin/mineral premix | 2g |
| Moisture | up to 23.5g |

The powder contained in the sachet is meant to be dissolved in a suitable liquid such as water prior to use, such as by tube feeding, to provide 1 kcal per ml, i.e dissolved in water and will normally be administered so that an amount of 2000 kcal is consumed.

### Example 4

Powder packed in sachet and comprising:

| Component | levels |
|---|---|
| Creatine | 2 grams |
| guanidino acetate | 1 grams |
| L-serine | 1 grams |
| folic acid | 200 µg |
| vitamin B12 | 3 µg |
| vitamin B6 | 1 mg |
| vitamin B2 | 0.85 mg |
| vitamin C | 120 mg |
| flavor premix | 1g |

The powder contained in the sachet is meant to be dissolved in a suitable 100 ml liquid such as water, juice, milk, pudding, or sauce for convenient administration.

### Example 5

Liquid formula for complete nutrition of a person that is at risk or suffering from decubitus that comprises per 100 ml:

| Component | levels |
|---|---|
| Caseinate (mix of sodium and calcium caseinates) | 4 g |
| L-serine | 0.3 g |
| L-histidine | 0.1 g |
| Guanidino acetate | 0.1 g |
| digestible carbohydrates (maltodextrin DH19) | 12.3 g |
| Lipids (mixture of vegetable oils) | 3.9 g |
| Vitamin premix 3 | 2.0 g |

### Example 6:

Liquid formula for perioperative use, comprising per 100 ml:

| Component | levels |
|---|---|
| L-serine | 4 g |
| digestible carbohydrates | 4 g |
| guanidino acetate | 0.5 g |
| folic acid | 200 µg |
| vitamin B12 | 4 µg |
| vitamin B6 | 2 mg |
| pantothenic acid | 10 mg |
| biotin | 1 mg |
| egg phospholipids | 2 g |
| olive oil, sunflower oil (50/50) | 2 g |
| flavor premix | 0.2 g |

### Example 7:

Infant formula for premature infants that comprises per 100 ml

| Component | levels |
|---|---|
| caseine | 0.4 g |
| whey | 0.6 g |
| L-serine | 0.2 g |
| lactose | 7.1 g |
| lipids | 3.6 g |
| guanidino acetate | 0.1 g |
| Na | 18 mg |
| K | 65 mg |
| Cl | 40 mg |
| Ca | 42 mg |
| P | 21 mg |
| Mg | 5 mg |
| Zn | 0.5 mg |
| Fe | 0.6 mg |
| Cu | 0.05 mg |
| I | 10 µg |
| Vitamin A | 80 µg RE |
| Vitamin D3 | 1.4 µg |
| Vitamin E | 0.8 mg TE |
| Vitamin K | 15 µg |
| Vitamin B1 | 40 µg |
| Vitamin B2 | 0.1 mg |
| Vitamin B3 | 0.75 mg |
| folic acid | 20 µg |
| pantothenic acid | 0.3 mg |
| Vitamin B6 | 60 µg |
| Vitamin B12 | 0.6 µg |
| biotine | 1.5 µg |
| Vitamin C | 7 mg |
| choline | 7 mg |
| taurine | 4.6 mg |

## Claims

1. A nutritional or pharmaceutical composition comprising:
a)-a protein fraction containing L-serine selected from the group consisting of L-serine, protein or peptides; and
b)-an energy metabolism precursor selected from glycocyamine (GA), equivalents thereof, and mixtures thereof,
by equivalents thereof, it is meant:
- compounds which provide GA in blood plasma after oral consumption and which are selected from guanidino-acetic acid salts of sodium, potassium, calcium, ammonium, magnesium, zinc, iron, copper, chromium and mixtures thereof and/or GA bidentate with zinc to a maximum of 100mg zinc per daily dose of the total composition,
- ester and/or ether compounds that can be metabolised to guanidino acetate in the gut or body, and compounds where the amidino group of the GA is modified by protonation and thus forms a salt,
wherein the composition is free of glycine or if glycine is present within the composition, the weight ratio of L-serine to glycine after hydrolysis of the composition is more than 2.7:1.

2. The composition according to claim 1, wherein the molar ratio of the energy metabolism precursor and the excess of L-serine versus glycine is in the range of 0.1:1 to 10:1.

3. The composition according to claims 1 or 2, wherein the composition further comprises creatine.

4. The composition according to claim 3, wherein the energy metabolism precursor and creatine are present within the composition in a weight ratio of at least 0.2:5.

5. The composition according to any one of the preceding claims, wherein the composition further comprises vitamin B6, vitamin B12 and/or folic acid.

6. Composition as defined in any one of the preceding claims for increasing the creatine response within mammals muscle and methylation reaction capacity of organisms.

7. Composition according to claim 6, wherein the energy metabolism precursor is administered at a daily dosage of at least 0.2 g up to 4 g.

8. Composition according to claim 6 or 7, wherein the composition is a nutritional or pharmaceutical supplement and wherein the protein fraction containing L-serine is administered at a daily dosage of at least 1 g.

9. Composition according to claim 6 or 7, wherein the composition is a complete nutrition or pharmaceutical composition and wherein the protein fraction containing L-serine is administered at a daily dosage of at least 4.8 g.

10. Composition according to any one of claims 6 - 9, wherein said composition is administrable to an athlete.

11. Composition according to any one of claims 6 - 9, wherein said composition is administrable to a disease-affected person, a vegetarian person or an elderly person.

12. Composition according to any one of claims 6 - 9 for the stimulation and/or increase of anabolic processes and/or the providing of an increase of the lean body mass, and/or prevention and/or treatment of muscle catabolism or even cachexia and/or for the improvement of the energy status of tissues and cells.

13. Composition according to any one of claims 6 - 9 for the prevention and/or treatment of disorders selected from cancer, neurological disorders, migraine, allergy, insulin resistance which improves glucose tolerance and decreases side effects of diabetes type II, cardiovascular and cerebrovascular disorders, hypercholesterolaemia, hypertension, subfertility, uncontrolled inflammation processes, pneumonia, and mixtures thereof, and/or prevention of hearing loss, and/or improvement of wound healing, and/or improvement of gut barrier function and/or prevention of sepsis.

## Patentansprüche

1. Nahrungs- oder pharmazeutische Zusammensetzung umfassend
a) eine Proteinfraktion enthaltend L-Serin ausgewählt aus der Gruppe bestehend aus L-Serin, Protein oder Peptiden und
b) eine aus Glycocyamin (GA), Äquivalenten davon und Mischungen davon ausgewählte Vorstufe des Energiestoffwechsels,
wobei unter Äquivalente davon zu verstehen ist:
Verbindungen, die nach oralem Verzehr im Blutplasma GA bereitstellen und aus Salzen der Guanidinoessigsäure von Natrium, Kalium,
Calcium, Ammonium, Magnesium, Zink, Eisen, Kupfer, Chrom und
Mischungen davon und/oder bidentat an Zink gebundenem GA bis zu einem Maximum von 100 mg Zink pro Tagesdosis der Gesamtzusammensetzung ausgewählt sind,
Ester und/oder Ether-Verbindungen, die im Darm oder im Körper zu Guanidinoacetat metabolisiert werden können und
Verbindungen, in denen die Amidino-Gruppe des GA durch Protonisierung modifiziert ist und daher ein Salz bildet,
wobei die Zusammensetzung frei von Glycin ist oder, falls in der Zusammensetzung Glycin vorliegt, das Gewichtsverhältnis von L-Serin zu Glycin nach Hydrolyse der Zusammensetzung nicht mehr als 2,7:1 beträgt.

2. Zusammensetzung nach Anspruch 1, wobei das molare Verhältnis der Vorstufe des Energiestoffwechsels und der Überschuss an L-Serin im Vergleich zu Glycin im Bereich von 0,1:1 bis 10:1 liegt.

3. Zusammensetzung nach den Ansprüchen 1 oder 2, wobei die Zusammensetzung weiterhin Kreatin enthält.

4. Zusammensetzung nach Anspruch 3, wobei die Vorstufe des Energiestoffwechsels und Kreatin in der Zusammensetzung in einem Gewichtsverhältnis von mindestens 0,2:5 vorliegen.

5. Zusammensetzung nach einem der vorhergehenden Ansprüche, wobei die Zusammensetzung weiterhin Vitamin B6, Vitamin B12 und/oder Folsäure enthält.

6. Zusammensetzung wie in einem der vorhergehenden Ansprüche definiert zur Erhöhung der Kreatinreaktion im Säugermuskel und der Leistungsfähigkeit von Organismen bei der Methylierungsreaktion.

7. Zusammensetzung nach Anspruch 6, wobei die Vorstufe des Energiestoffwechsels in einer Tagesdosis von mindestens 0,2 g bis 4 g verabreicht wird.

8. Zusammensetzung nach Anspruch 6 oder 7, wobei die Zusammensetzung ein Nahrungs- oder pharmazeutisches Ergänzungsmittel ist und wobei die L-Serin enthaltende Proteinfraktion in einer Tagesdosis von mindestens 1 g verabreicht wird.

9. Zusammensetzung nach Anspruch 6 oder 7, wobei die Zusammensetzung eine vollständige Nahrungs- oder pharmazeutische Zusammensetzung ist und
wobei die L-Serin enthaltende Proteinfraktion in einer Tagesdosis von mindestens 4,8 g verabreicht wird.

10. Zusammensetzung nach einem der Ansprüche 6 bis 9, wobei die Zusammensetzung an Sportler verabreichbar ist.

11. Zusammensetzung nach einem der Ansprüche 6 bis 9, wobei die Zusammensetzung an einen von einer Krankheit betroffenen Menschen, einen Vegetarier oder einen älteren Menschen verabreichbar ist.

12. Zusammensetzung nach einem der Ansprüche 6 bis 9 zur Anregung und/oder Erhöhung anabolischer Prozesse und/oder zur Bereitstellung einer Erhöhung der Körpermasse bei einem mageren Körper und/oder zur Verhütung und/oder Behandlung des Abbaustoffwechsels der Muskeln oder sogar der Kachexie und/oder zur Verbesserung des Energiestatus von Geweben und Zellen.

13. Zusammensetzung nach einem der Ansprüche 6 bis 9 zur Verhütung und/oder Behandlung von aus Krebs, neurologischen Störungen, Migräne, Allergie, Insulinresistenz, die die Glucose-Toleranz verbessert und die Nebenwirkungen von Diabetes-Typ II vermindert, kardiovaskulären und cerebrovaskulären Störungen, Hypercholesterinämie, Hypertonie, verminderter Fertilität, unkontrollierten Entzündungsprozessen, Lungenentzündung und Mischungen davon ausgewählten Störungen und/oder zur Verhütung von Hörverlust und/oder zur Verbesserung der Wundheilung und/oder der Barrierefunktion des Darms und/oder zur Verhütung der Sepsis.

## Revendications

1. Composition nutritionnelle ou pharmaceutique comprenant :
a) une fraction protéique contenant de la L-sérine choisie dans le groupe consistant en la L-sérine, une protéine ou les peptides ; et
b) un précurseur du métabolisme énergétique choisi parmi la glycocyamine (GA), ses équivalents, et leurs mélanges,
par ses équivalents, on entend :
- les composés qui apportent la GA dans le plasma sanguin après consommation orale et qui sont choisis parmi les sels d'acide guanidino-acétique et de sodium, potassium, calcium, ammonium, magnésium, zinc, fer, cuivre, chrome et leurs mélanges et/ou un bidenté de GA avec le zinc jusqu'à un maximum de 100 mg de zinc par dose journalière de la composition totale,
- les composés esters et/ou éthers qui peuvent être métabolisés en guanidino acétate dans l'intestin ou le corps, et
- les composés où le groupe amidino de la GA est modifié par protonation et forme ainsi un sel,
où la composition est dépourvue de glycine ou, si de la glycine est présente dans la composition, le rapport en poids de la L-sérine à la glycine après hydrolyse de la composition est supérieur à 2,7 : 1.

2. Composition selon la revendication 1 où le rapport molaire du précurseur du métabolisme énergétique et de l'excès de L-sérine par rapport à la glycine est dans la plage de 0,1 : 1 à 10 : 1.

3. Composition selon la revendication 1 ou 2 où la composition comprend en outre de la créatine.

4. Composition selon la revendication 3 où le précurseur du métabolisme énergétique et la créatine sont présents dans la composition dans un rapport en poids d'au moins 0,2 : 5.

5. Composition selon l'une quelconque des revendications précédentes où la composition comprend en outre de la vitamine B6, de la vitamine B12 et/ou de l'acide folique.

6. Composition selon l'une quelconque des revendications précédentes pour augmenter la réponse créatinique dans un muscle de mammifère et la capacité de réaction de méthylation d'organismes.

7. Composition selon la revendication 6 où le précurseur du métabolisme énergétique est administré à une posologie journalière d'au moins 0,2 g jusqu'à 4 g.

8. Composition selon la revendication 6 ou 7 où la composition est un complément nutritionnel ou pharmaceutique et où la fraction protéique contenant de la L-sérine est administrée à une posologie journalière d'au moins 1 g.

9. Composition selon la revendication 6 ou 7 où la composition est une composition nutritionnelle ou pharmaceutique complète et où la fraction protéique contenant de la L-sérine est administrée à une posologie journalière d'au moins 4,8 g.

10. Composition selon l'une quelconque des revendications 6 - 9 où ladite composition est administrable à un athlète.

11. Composition selon l'une quelconque des revendications 6 - 9 où ladite composition est administrable à une personne affectée par une maladie, une personne végétarienne ou une personne âgée.

12. Composition selon l'une quelconque des revendications 6 - 9 pour la stimulation et/ou l'augmentation des processus anaboliques et/ou la fourniture d'une augmentation de la masse corporelle maigre, et/ou la prévention et/ou le traitement du catabolisme musculaire ou même de la cachexie et/ou pour l'amélioration du bilan énergétique de tissus et cellules.

13. Composition selon l'une quelconque des revendications 6 - 9 pour la prévention et/ou le traitement de troubles choisis parmi le cancer, les troubles neurologiques, la migraine, l'allergie, la résistance à l'insuline qui améliore la tolérance au glucose et réduit les effets secondaires du diabète de type II, les troubles cardiovasculaires et cérébrovasculaires, l'hypercholestérolémie, l'hypertension, la subfécondité, les processus inflammatoires incontrôlés, la pneumonie, et leurs mélanges, et/ou la prévention des pertes de l'audition, et/ou l'amélioration de la cicatrisation des plaies, et/ou l'amélioration de la fonction de barrière intestinale et/ou la prévention de la septicémie.
